Europäisches Patentamt

European Patent Office

Office européen des brevets

(19)

(11) Publication number: 0 333 442

A1

# (12) EUROPEAN PATENT APPLICATION

(21) Application number: 89302515.5

(22) Date of filing: 14.03.89

(51) Int. Cl.⁴: A61B 5/02

(30) Priority: 14.03.88 JP 33444/88 U

(43) Date of publication of application:
20.09.89 Bulletin 89/38

(84) Designated Contracting States:
DE FR GB IT NL SE

(71) Applicant: COLIN ELECTRONICS CO., LTD.
2007-1, Hayashi
Komaki-shi Aichi-ken(JP)

(72) Inventor: Kaida, Noriyuki c/o Colin Electronics
Company ltd.
2007-1, Hayashi Komaki-shi
Aichi-ken(JP)

(74) Representative: Senior, Alan Murray et al
J.A. KEMP & CO 14 South Square Gray's Inn
London WC1R 5EU(GB)

(54) Pulse wave detecting apparatus.

(57) An apparatus for detecting pulse wave produced from an arterial vessel (34) of a subject, including a main body (12, 52, 62), a band member (10) connected at both ends thereof to the main body, the main body being set on a body portion (20) of the subject by fastening the band member around the body portion, such that the main body is held in contact with the body portion, and a pulse wave sensor (14) supported by the main body, for detecting the pulse wave and generating electric signal representing and detected pulse wave, the main body including a plurality of pivotable members (16, 18, 19; 54, 58; 64a-64f, 18, 19) which are pivotably connected to each other, each of the pivotable members including a contact portion (16a, 18a, 19a; 54a, 58a; 65a-65f, 18, 19a) which contacts the body portion while the main body is set on the body portion.

FIG. I

EP 0 333 442 A1

## PULSE WAVE DETECTING APPARATUS

The present invention relates to a pulse wave detecting apparatus which is set around a wrist or other body member of a subject with a fastening band.

There is known a pulse wave detecting apparatus having (a) a housing in which a pulse wave sensor is accommodated, (b) a wrist band for fastening the housing around a wrist of a subject such that the pulse wave sensor is located in position right above an arterial vessel extending through the wrist, and (c) pressing means for pressing the pulse wave sensor against the arterial vessel with a suitable pressing force so as to detect pulse wave, i.e., oscillatory pressure wave produced by expansion and contraction of the arterial vessel synchronous with heartbeat of the subject.

However, in the above-indicated conventional apparatus, the wrist band is formed of a material with a comparatively high flexibility, such as plastic, fabric and rubber, accordingly the housing may be displaced along a surface of the wrist even after the housing is set on the wrist. In such case the pulse wave sensor accommodated in the housing is also displaced out of position above the arterial vessel, and therefore the accuracy of detection of the pulse wave is deteriorated.

It is therefore an aim of the present invention to provide a pulse wave detecting apparatus which is fixed with stability on a wrist or other body member of a subject and thereby permits the pulse wave sensor to be located with accuracy at a position right above an arterial vessel of the subject.

According to the present invention there is provided an apparatus for detecting blood conditions in an artery of a subject, comprising:
a main part;
a band member connected at both ends thereof to said main part, said main part being in use set on a body portion of the subject by fastening said band member around said body portion, such that said man part is held in contact with said body member; and
a sensor to be positioned on said body portion above said artery and supported by said main part; and for generating a signal representing the detected condition, characterised in that
said main part comprises a plurality of pivotable members which are pivotably connected to each other and contacts said body portion when said main part is set on said body portion.

The sensor supported by said main part may be a pulse wave sensor for detecting pulse waves in an arterial vessel in said body portion.

In the apparatus constructed as described above, the main body includes the plurality of pivotable members each having the contact portion which contact a body member of a subject while the main part is set on the body member. In the case where the present apparatus is used on, for example, a wrist of the subject, the pivotable members closely contact a protuberance of the wrist corresponding to a radius. Stated differently, the pivotable members firmly grasp or sandwich the radius via the wrist surface (skin) over the radius, as a result that the band member is fastened around the wrist. Thus, the main part and the pulse wave sensor supported thereby are protected from being displaced out of position along the wrist surface, once the main part is fixed on the wrist with the band member fastened around the wrist. It is preferred that the pivotable members have suitable dimensions for the main part as a whole to be effectively protected from being displaced out of position, that is, to firmly grasp the radius. Thus, the present apparatus is fixed with satisfactory stability on the body member of the subject thereby permitting the position of the pulse wave sensor to be maintained with accuracy.

In a preferred embodiment of the apparatus of the present invention, the main part comprises a housing which said sensor is accommodated, and a pair of links pivotably connected to opposite ends of said housing, respectively, said band member being connected at its ends to said links said pivotable members comprising said housing and said pair of links.

In an alternative preferred embodiment of the present invention the main part comprises a series of unit members which are pivotably connected to each other such that said series of unit members define a variable space, said band member being connected at its ends to end unit members of said series of unit members, and a housing in which said sensor is accommodated, said housing being accommodated in said variable space such that said housing is rotatably supported by said series of unit members, said pivotable members comprising said series of unit members.

According to a feature of this embodiment the pivotable radius comprise a pair of links pivotably connected to said end unit members of said series of unit members, respectively, said band member being directly connected at both ends thereof to said pair of links.

According to a preferred feature of the above embodiments of the invention, the apparatus further comprises drive means for moving said sensor in a crossing direction substantially perpendicular to the artery so as to locate said sensor in a desired position, said drive means being supported by said main part.

It is also preferred that the drive means comprise an externally threaded screw bolt engaged with an internally threaded portion of said sensor, and an electric motor for rotating said screw bolt so as to move said sensor in said crossing direction.

In another embodiment of the apparatus of the invention, the pulse wave sensor comprises a pressure sensitive semiconductor element which is pressed against the artery vessel via a body surface of the body portion of the subject, and pressing means for pressing the semiconductor element against the artery via the body surface.

According to a preferred feature of the above embodiment of the invention, the pulse wave sensor comprises a casing in which the semiconductor element is accommodated, the pressing means including an elastic diaphragm disposed in the casing such that the semiconductor element is supported by the diaphragm and that the diaphragm cooperates with the casing to define a fluid-tight space in the casing, the semiconductor element being moved toward the body surface of the body portion when the diaphragm is expanded upon supply of pressurized fluid to the fluid-tight space.

In yet another embodiment of the apparatus of the invention, the main body comprises a first housing in which said sensor is accommodated, and a second housing pivotably connected to said first housing, said band member being connected at one of said both ends thereof to said first housing and at the other end thereof to said second housing, said pivotable members comprising said first and second housings.

According to a preferred feature of the above embodiment of the invention, the apparatus further comprises drive means for moving said sensor in a crossing direction substantially perpendicular to the artery of said body portion so as to locate said sensor at a desired position.

It is preferred that the drive means comprise means for moving said sensor in a crossing direction substantially perpendicular to the artery so as to locate said sensor in a desired position, said drive means being supported by said main part.

The invention will be further described hereinafter with reference to the following detailed description of exemplary embodiments and the accompanying drawings, in which:

Fig. 1 is a front elevational view showing a pulse wave detecting apparatus of the present invention set around a wrist of a subject with a part of the apparatus removed;

Fig. 2 is a perspective view of the apparatus of Fig. 1;

Fig. 3 is a longitudinal cross sectional view of a part of the apparatus of Fig. 1;

Fig. 4 is a front elevational view showing another pulse wave detecting apparatus of the invention with a part thereof removed;

Fig. 5 is a front elevational view of yet another pulse wave detecting apparatus of the invention; and

Fig. 6 is a front elevational view showing the apparatus of Fig. 5 with a part thereof removed.

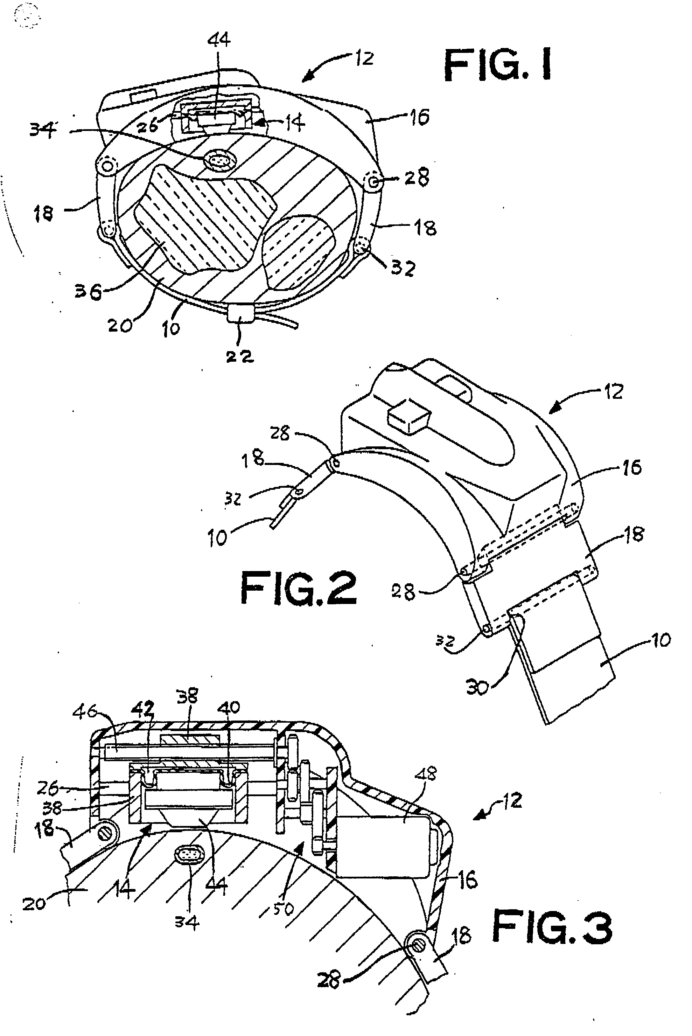

Referring first to Fig. 1, there is shown a pulse wave detecting apparatus embodying the present invention, the apparatus being set around a wrist 20 of a subject. The apparatus includes a wrist band 10 and a main body 12. The wrist band 10 includes a pair of half belts 10a, 10b and an engaging member 22 for engaging the pair of belts with each other. The half belts 10a, 10b are formed of a material with a comparatively high flexibility, such as plastic, fabric or rubber.

The main body 12 includes a housing 16 in which a pulse wave sensor 14 is accommodated, and a pair of links 18, 19 pivotably connected to longitudinally opposite ends of the housing 14, respectively. The housing and links 14, 18, 19 are formed of a material with a comparatively high rigidity, such as metal, engineering plastic or synthetic resin. The wrist band 10 (i.e., half bands 10a, 10b) is connected at each end to one of the pair of links 18, 19. The main body 12 is fixed on the wrist 20 by winding the wrist band 10 around the wrist 20 and engaging the half bands 10a, 10b with each other with the engaging member 22. With the main body 12 fixed on the wrist 20, an inner surface 16a of the housing 16 is held in contact with a surface 20a of the wrist 20 and an opening 17 (Fig. 3) of the housing 16 is opposed to the wrist surface 20a, while inner surfaces 18a, 19a of the pair of links 18, 19 are also held in contact with the wrist surface 20a.

The pulse wave sensor 14 is accommodated in the housing 16 such that the sensor 14 is movable along a pair of guide grooves 26, 26 in a crossing direction substantially perpendicular to a radial artery 34 from which pulse wave is detected by the present apparatus. The pair of guide grooves 26, 26 are formed in longitudinal side walls of the housing 16.

As shown in phantom line in Fig. 2, the pair of links 18, 19 are pivotably connected to the pair of longitudinal opposite ends of the housing 16, via a pair of first connecting pins 28, 28, respectively. The first connecting pins 28, 28 extend through the corresponding links 18, 19 and the corresponding ends of the housing 16 in a direction across the main body 12. Thus, the housing and links 16, 18, 19 are pivotably connected to each other.

Each of the pair of links 18, 19 has a rectangular recess 30. Each of the pair of half belts 10a, 10b has a passage at one of both ends thereof, the other ends of the pair of half belts 10a, 10b being engaged with each other with the engaging member 22. The half belts 10a, 10b are connected to the corresponding links 18, 19 via a pair of second connecting pins 32, 32 which extend through the corresponding passages of the half belts 10a, 10b and the corresponding links 18, 19 (and corresponding recesses 30), 30 in the direction of width of the main body 12. The passage of each of the half belts 10a, 10b is formed by folding back an end portion thereof and sewing the folded potion to an adjacent portion thereof. A length of one of the pair of links 18, as measured in a direction of length of the main body 12, is predetermined such that the link 18 closely contacts, and is effectively pressed against, a protuberance 21 of the wrist 20 corresponding to a radius 36 when the main body 12 is fixed on the wrist 20 with the wrist band 10 wound and fastened around the wrist 20. Similarly the length of the housing 16 as measured in the same direction is so predetermined as to firmly grasp the radius 36 in cooperation with the link 18.

As is apparent from the foregoing, in the present embodiment, the housing 16 and the link 18 cooperate with each other to grasp or sandwich the radius 36 via the wirst surface 20a as a result that the link 18 is pivoted relative to the housing 16 so as to have an appropriate angle therebetween, when the main body 12 is fixed on the wrist 20 with the wrist band 10 wound and fastened around the wrist 20. The comparatively rigid housing and link 16, 18 closely contact, and are effectively pressed against, the wrist 20. The main body 12 is firmly fixed on the wrist 20, whereby the pulse wave sensor 14 is located in position right above the radial artery 34. In such condition, the main body 12 is well protected from being displaced out of the position at which the main body 12 (housing and link 16, 18) grasps the radius 36. Consequently the main body 12 is fixed with stability on the wrist 20, assuring that the pulse wave sensor 14 detects accurate pulse wave from the radial artery 34.

As is shown in Fig. 3, the pulse wave sensor 14 includes a casing 38, an elastic diaphragm 40 and a pressure-sensitive element 44. The diaphragm 40 is disposed in the casing such that the diaphragm 40 cooperates with the casing 38 to define an air-tight space 42 in the casing 38, and that the pressure-sensitive element 44 is supported by the diaphragm 40. The pressure-sensitive element 44 has a silicon chip (not shown) on a contact surface thereof which contacts the wrist surface 20a, and a plurality of semiconductor strain gauges (e.g., pressure-sensitive resistances) (not shown) provided on the silicon chip. The strain gauges are arranged in a row along a straight line parallel to the above-indicated crossing direction in which the pulse wave sensor 14 is moved.

An externally threaded screw bolt 46 is disposed in, and supported by, the housing 16 such that the screw bolt 46 is engaged with an internally threaded portion of the casing 38 of the pulse wave sensor 14. In the housing 16 also are disposed an electric motor 48 for rotating the screw bolt 46, and a reduction gear unit 50 for transmitting reduced rotating force of the electric motor 48 to the screw bolt 46. The bolt screw 46, electric motor 48 and gear unit 50 cooperate with each other to serve as the drive means for moving the pulse wave sensor 14 in the crossing direction so as to locate the sensor 14 in position right above the radial artery 34.

Pulse wave detection is effected by the present appratus, as follows: first, the pulse wave sensor 14 is moved to a position right above the radial artery 34, and subsequently pressure in the air-tight space 42 of the pulse wave sensor 14 is increased so as to press the pressure- sensitive element 44 against the radial artery 34 via the wrist surface 20a with an optimum pressing force. The pressure in the air-tight space 42 is varied as a result of the regulation of supply of pressurized air thereto from an air pump (not shown). The pressure-sensitive element 44 detects pulse wave produced from the radial artery 34 and generates electric signal representing the detected pulse wave. Since pulse wave reflects variation in blood pressure of the subject, the electric signal supplied from the element 44 is utilized by a control means (not shown) for monitoring blood pressure of the subject.

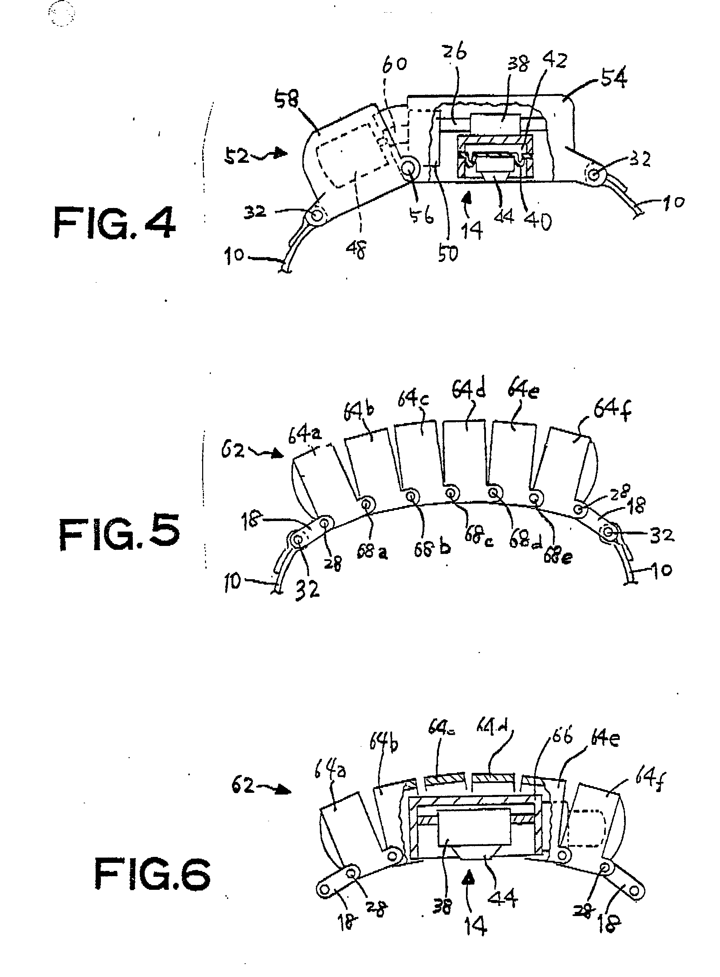

Referring next to Fig. 4 there is shown another pulse wave detecting apparatus embodying the present invention. The same refrence numerals as used in Figs. 1-3, are used in Fig. 4 to designate corresponding elements or parts, and repetitive description of those elements and parts is omitted.

In this embodiment, the main body 52 includes a first housing 54 in which a pulse wave sensor 14 is accommodated, and a second housing 58 pivotably connected to the first housing 54 with a connecting pin 56. The first and second housings 54, 58 are formed of a material with a comparatively high rigidity, similar to the housing 16 of Fig. 1, and are set on the wrist 20 such that openings of the first and second housings 54, 58 are opposed to the wrist surface 20a. In this embodiment, a wrist band 10, namely, a pair of half belts 10a, 10b are connected directly to the first and second housings 54, 58, respectively. However, it is possible to indirectly connect the wrist band 10 to the first and second housings 54, 58 via, for example, a pair of links like the links 18, 19 of Fig. 1.

An electric motor 48 is accommodated in the second housing 58, while in the first housing 54 are disposed, in addition to the pulse wave sensor 14, an externally threaded screw bolt 46 engaged with an internally threaded portion of the sensor 14 and a reduction gear unit 50. A flexible coupling 60 is provided between the first and second housings 54, 58 to connect an output shaft of the electric motor 48 to the reduction gear unit 50. The flexible coupling 60 is constituted by a coil spring, or is formed of a resilient material such as plastic. Irrespective of what angle is contained between the first and second housings 54, 58, rotating force of the motor 48 is transmitted to the screw bolt 46 via the flexible coupling 60 and the gear unit 50, so as to move the pulse wave sensor 14 in a crossing direction substantially perpendicular to the radial artery 34 and located at a position above the artery 34.

In the instant embodiment, the first and second housings 54, 58 are pivotably connected to each other. Accordingly, when the wrist band 10 is wound and fastened around the wrist 20 so that the main body 52 is fixed on the wrist 20, the first and second housings 54, 58 sandwich therebetween the protuberance of the wrist 20 corresponding to the radius 36 as a result that the second housing 58 is pivoted relative to the first housing 54, whereby the first and second housings 54, 58 are prevented from being displaced along the wrist surface 20a. Thus, the main body 52 is fixed with stability on the wrist 20, and the pulse wave sensor 14 is located and retained with stability at the position right above the radial artery. Thus, the instant apparatus assures the accuracy of detection of pulse wave.

Referring further to Figs. 5 and 6 there is shown a further pulse wave detecting apparatus embodying the present invention. A main body 62 is constituted by a series of hollow unit members 64a, 64b, ..., 64f which are pivotably connected to each other with connecting pins 68a, 68b, ..., 68e. Each of the hollow members 64 has a generally U-shaped cross section. The series of hollow members 64 define a space 65 whose volume is variable when the hollow members 64 are pivoted relative to each other. The main body 62 is set on the wrist 20 such that an opening of the hollow members 64 is opposed to the wrist surface 20a. A housing 66 is accommodated in the variable space 65 such that the housing 66 is rotatable. Specifically described, the housing 66 is supported by the middle connecting pin 68c so as to be rotatable relative to the hollow members 64. This arragement assures that the housing 66 closely contact the wrist surface 20a when the main body 62 is fixed on the wrist 20.

As shown in Fig. 6, a pulse wave sensor 14 is accommodated in the housing 66 such that the sensor 14 is movable in a direction of length of the main body 62. An externally threaded screw bolt 46 is engaged with an internally threaded portion of a casing 38 of the pulse wave sensor 14. A reduction gear unit 50 and an electric motor 48 are supported by the housing 66. Rotating force of the motor 48 is transmitted to the screw bolt 46 via the gear unit 50 so as to move the pulse wave sensor 14.

In the present embodiment, the hollow unit members 64a, 64b, ..., 64f are pivotably connected to each other. Accordingly, when the wrist band 10 is wound and fastened around the wrist 20 so that the main body 62 is fixed on the wrist 20, the hollow members 64 grasp the protuberance of the wrist 20 corresponding to the radius 36, whereby the hollow members 64 are protected from being displaced along the wrist surface 20a. Thus, the main body 62 is fixed with stability on the wrist 20, and the pulse wave sensor 14 is located and maintained in position right above the radial artery 34, whereby pulse wave is detected with accuracy.

While the present invention has been described in its preferred embodimets, the invention may be embodied with various modifications.

While in the first embodiment of Fig. 1 the pair of links 18, 19 are used, it is possible to omit the link 19 but not the link 18, which is pressed against the radius 36 when the main body 12 is fixed on the wrist 20 with the wrist band 10 fastened around the wrist 20.

Although in the embodiment of Figs. 5 and 6 the pair of links 18, 19 are provided between the wrist band 10 and the main body 62 to connect the members 10, 62 to each other, it is possible to omit the links 18, 19 in the embodiment.

## Claims

1. An apparatus for detecting blood conditions in an artery (34) of a subject, comprising:
a main part (12, 52, 62);
a band member (10) connected at both ends thereof to said main part, said main part being in use set on a body portion (20) of the subject by fastening said band member around said body portion, such that said man part is held in contact with said body member; and
a sensor (14) to be positioned on said body portion above said artery and supported by said main part; and for generating a signal representing the detected condition, characterised in that
said main part comprises a plurality of pivotable members (16,18, 19; 54, 48; 64a-64f, 18, 19) which are pivotably connected to each other and contacts said body portion when said main part is set on said body portion.

2. The apparatus as set forth in claim 1, further comprising
drive means (46, 46, 50) for moving said sensor (14) in a crossing direction substantially perpendicular to the artery (34) so as to locate said sensor in a desired position, said drive means being supported by said main part.

3. The apparatus as set forth in claim 2, wherein said drive means comprises
an externally threaded screw bolt (46) engaged with an internally threaded portion (38) of said sensor (14), and
an electric motor (48) for rotating said screw bolt so as to move said sensor in said crossing direction.

4. The apparatus as set forth in claim 1, 2 or 3 wherein said main part (12) comprises
a housing (16) in which said sensor (14) is accommodated, and
a pair of links (18, 19) pivotably connected to opposite ends of said housing, respectively, said band member (10) being connected at its end to said links
said pivotable members comprising said housing and said pair of links.

5. The apparatus as set forth in claim 1, 2 or 3 wherein said main part (62) comprises
a series of unit members (64a-64f) which are pivotably connected to each other such that said series of unit members define a variable space (67), said band member being connected at its ends to end unit members of said series of unit members, and
a housing (66) in which said sensor (14) is accommodated, said housing being accommodated in said variable space such that said housing is rotatably supported by said series of unit members,
said pivotable members comprising said series of unit members.

6. The apparatus as set forth in claim 5, wherein said pivotable members comprise a pair of links (18, 19) pivotably connected to said end unit members (64a, 64f) of said series of unit members (64a-64f), respectively, said band member (10) being directly connected at both ends thereof to said pair of links.

7. The apparatus as set forth in claim 1, wherein said main part (52) comprises
a first housing (54) in which said sensor (14) is accommodated, and
a second housing (58) pivotably connected to said first housing,
said band member (10) being connected at one of said both ends thereof to said first housing and at the other end thereof to said second housing,
said pivotable members comprising said first and second housings.

8. The apparatus as set forth in claim 7, further comprising
drive means (46, 48, 50, 60) for moving said sensor (14) in a crossing direction substantially perpendicular to the artery of said body portion so as to locate said sensor at a desired position.

9. The apparatus as set forth in claim 8, wherein said drive means comprises
an electric motor (48) accommodated in said second housing (58),
an externally threaded screw bolt (46) accommodated in said first housing (54) and engaged with an internally threaded portion (38) of said sensor (14), and
a flexible coupling (60) for transmitting rotating force of said electric motor to said screw bolt.

10. An apparatus according to any preceding claim wherein said sensor is a pulse wave sensor (14) for detecting pulse waves in an arterial vessel (34) in said body portion.

11. An apparatus according to claim 10 wherein said pulse wave sensor (14) comprises
a pressure-sensitive semiconductor element (44) which in use is pressed against said arterial vessel (34) via a body surface (20a) of said body portion (20) of the subject, and
pressing means (40) for pressing said semiconductor element against said arterial vessel via said body surface.

12. The apparatus as set forth in claim 11, wherein said pulse wave sensor (14) comprises
a casing (38) in which said seimiconductor element (44) is accommodated,
said pressing means including an elastic diaphragm (40) disposed in said casing such that said semiconductor element is supported by said diaphragm and that said diaphragm cooperates with said casing to define a fluid-tight space (42) in said casing, said semiconductor element being moved toward said body surface (20a) of said body portion (20) when said diaphragm is expanded upon supply of pressurized fluid to said fluid-tight space.

FIG. 1

FIG. 2

FIG. 3

FIG.4

FIG.5

FIG.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-2 118 719 (R.L. BROADWATER et al.)<br>* page 2, line 79 - page 3, line 109; figures 1-4 * | 1,2,4,8,10 | A 61 B 5/02 |
| A | | 5,11 | |
| A | US-A-4 409 983 (D.E. ALBERT)<br>* abstract; column 2, line 38 - column 3, line 4; figures 1,2,3,7 * | 1,2,4,5,8,10,11 | |
| A | GB-A-2 070 775 (J.E. DRISCOLL)<br>* abstract and figure 1 * | 1 | |

TECHNICAL FIELDS SEARCHED (Int. Cl.4)

A 61 B    5/00
G 04 G    9/00

The present search report has been drawn up for all claims

| Place of search | Date of completion of the search | Examiner |
|---|---|---|
| BERLIN | 10-05-1989 | WEIHS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)